(19) Europäisches Patentamt
European Patent Office
Office européen des brevets

(11) Veröffentlichungsnummer: **0 045 861**
**B1**

(12) # EUROPÄISCHE PATENTSCHRIFT

(45) Veröffentlichungstag der Patentschrift: **03.10.84**

(21) Anmeldenummer: **81105612.6**

(22) Anmeldetag: **17.07.81**

(51) Int. Cl.³: **C 07 C 49/203,** C 07 C 45/68, C 11 B 9/00, C 07 C 33/02, C 07 C 29/136, C 07 C 29/40, C 07 C 131/00

(54) **Neue ungesättigte Verbindungen, (I), Verfahren zu deren Herstellung, Verwendung von I als Riechstoffe sowie Riechstoffkompositionen mit einem Gehalt an I.**

(30) Priorität: **08.08.80 CH 6019/80**
**15.06.81 CH 3913/81**

(43) Veröffentlichungstag der Anmeldung: **17.02.82 Patentblatt 82/7**

(45) Bekanntmachung des Hinweises auf die Patenterteilung: **03.10.84 Patentblatt 84/40**

(84) Benannte Vertragsstaaten: **CH DE FR GB LI NL**

(56) Entgegenhaltungen:
**DE - A - 2 363 535**
**DE - A - 2 432 351**
**FR - A - 1 305 994**

(73) Patentinhaber: **L. GIVAUDAN & CIE Société Anonyme, CH-1214 Vernier-Genève (CH)**

(72) Erfinder: **Ochsner, Paul Albert, Dr., 30 Avenue des Tilleuls, CH-1203 Genf (CH)**

(74) Vertreter: **Urech, Peter, Dr. et al, Grenzacherstrasse 124 Postfach 3255, CH-4002 Basel (CH)**

**EP 0 045 861 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Riechstoffe. Es handelt sich dabei um die Verbindungen der Formel

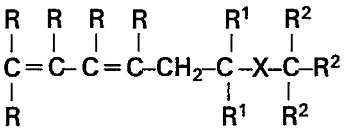

I

worin R, $R^1$ une $R^2$ Wasserstoff oder $C_{1-3}$-Alkyl darstellen, und X für 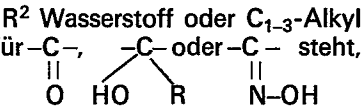 steht,

wobei, wenn beide Symbole $R^1$ Wasserstoff sind, alle drei Symbole $R^2$ $C_{1-3}$-Alkyl darstellen, und wobei die Gesamtzahl der Kohlenstoffatome im Molekül 15 nicht überschreitet.

Die Formel I soll die durch cis- bzw. trans-Konfiguration an den C=C und C=N Doppelbindungen möglichen Stereoisomeren umfassen.

Die einzelnen Symbole, R, $R^1$ und $R^2$ können gleich oder verschieden sein.

Als $C_{1-3}$-Alkyl-Reste können Methyl, Äthyl, n-Propyl, Isopropyl figurieren. Bevorzugt ist Methyl.

Die Erfindung betrifft ferner ein Verfahren zur Herstellung der Verbindungen I.

Dieses Verfahren ist dadurch gekennzeichnet, dass man eine Verbindung der Formel

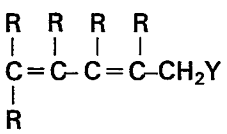

II

mit einer Verbindung der Formel

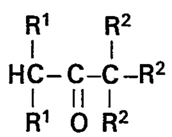

III

worin R, $R^1$ und $R^2$ obige Bedeutung besitzen und Y Halogen darstellt, in Anwesenheit einer Base umsetzt, und gegebenenfalls ein erhaltenes Keton der Formel I

a) zu einem sekundären Alkohol I reduziert oder b) durch Umsetzung mit einer Verbindung der Formel R Mg Y in einen tertiären Alkohol I überführt oder c) durch Umsetzung mit Hydroxylamin in ein Oxim I überführt.

Die Umsetzung der Verbindung der Formel II mit der Verbindung III kann nach an sich bekannten Methoden durchgeführt werden.

Als Halogenid II kann das Chlorid, Bromid oder Jodid dienen. Bevorzugt ist das Chlorid.

Die Umsetzung erfolgt vorzugsweise in Gegenwart einer starken anorganischen Base, wie z.B. einem Alkalimetallhydroxid, z.B. KOH, einem Erdalkalihydroxid, z.B. $Ca(OH)_2$, einem Alkalimetallamid, z.B. $NaNH_2$, oder einem Alkalimetallhydrid, z.B. NaH, oder aber auch einer organischen Base, wie z.B. Kalium-tert.butoxid.

Man kann mit oder ohne Zusatz eines Lösungsmittels arbeiten. Geeignete Lösungsmittel sind insbesondere aprotische bzw. wenig polare Lösungsmittel, z.B. Dimethoxymethan, Dimethoxyäthan, Diäthyläther oder Tetrahydrofuran oder aber auch Toluol oder Benzol.

Die Reaktionstemperatur liegt zweckmässigerweise zwischen ungefähr −20 und +50 °C, vorzugsweise zwischen −10 und +20 °C. Tiefere Temperaturen sind industriell unpraktisch, höhere Temperaturen könnten leicht zur Bildung unerwünschter polymerer Nebenprodukte führen.

Die Umsetzung des primär erhaltenen Ketons I mit der Verbindung R Mg Y erfolgt zweckmässigerweise nach den an sich bekannten Methoden der Grignardreaktion, siehe z.B. Organikum, Organisch-chemisches Grundpraktikum, Autorenkollektiv, 7. Auflage VEB Deutscher Verlag der Wissenschaften Berlin 1967, 486 seq.

Man arbeitet also zweckmässigerweise in Diäthyläther als Lösungsmittel und bei Temperaturen zwischen 0 °C und der Siedetemperatur des Äthers.

Als Halogenide kommen die üblichen Halogenide in Frage, bevorzugt ist aber das Bromid.

Das primär erhaltene Keton I kann vorzugsweise mittels Reduktionsmitteln wie Natriumborhydrid, Lithiumaluminiumhydrid, Triisopropylaluminat, etc. zum sekundären Alkohol reduziert werden. (L.F. Fieser & M. Fieser, Reagents for Organic Synthesis, John Wiley & Sons (1967), 581, 582, 584, 1049 und Organikum, loc. cit. Seiten 475–476.)Als Lösungsmittel kommen demgemäss insbesondere in Frage: Äthanol bzw. Wasser im Falle von $NaBH_4$, Diäthyläther im Falle von $LiAlH_4$, Isopropanol im Falle von Triisopropylaluminat.

Andererseits kann das primär erhaltene Keton I durch Umsetzung mit Hydroxylamin in das entsprechende Oxim übergeführt werden. Die Umsetzung erfolgt zweckmässigerweise nach an sich bekannten Methoden, siehe z.B. Organikum, Organisch-chemisches Grundpraktikum, Autorenkollektiv; 7.Auflage; VEB Deutscher Verlag der Wissenschaften; Berlin 1967, 375: Man lässt das Oxim zweckmässigerweise als Salz, z.B. als Hydrochlorid oder Sulfat in pyridinenthaltender alkalischer Lösung bzw. in wässrig-alkalischer Lösung mit dem Keton I reagieren; die Reaktionstemperatur ist dabei vorzugsweise die Rückflusstemperatur des Reaktionsgemisches.

Die Verbindungen I weisen besondere organoleptische Eigenschaften auf, auf Grund derer sie sich vorzüglich als Riechstoffe eignen.

Die Erfindung betrifft demgemäss auch die Verwendung der Verbindungen I als Riechstoffe.

Die Verbindungen der Formel I erinnern geruchlich im allgemeinen an Noten von Bergamotteöl, Lavendel, Salbei, Zitronenschalen; sie eignen sich aufgrund ihrer natürlichen Geruchsnoten und ihrer Haftdauer (Langzeiteffekt, insbesondere bezüglich Frische) insbesondere zur Modifizierung von bekannten, z.B.

α) blumigen Kompositionen, in denen z.B. die

Citrusnoten verstärkt zum Ausdruck kommen sollen (z.B. für Cologne-Typen u.ä., Extraits),

β) des weiteren aber auch von fruchtigen, z.B. vom Typ Johannisbeere (Extrait-Typen, Komposition der femininen wie der männlichen Richtung), von

γ) Kompositionen mit grünen Noten, wo insbesondere ein erwünschter natürlicher Effekt erzielt wird, und schliesslich von

δ) Kompositionen vom Typ agreste (also Kompositionen mit «ländlichen Noten»).

Besonders interessante Verbindungen sind:

2,4,4-Trimethyl-6,8-nonadien-3-on:
sehr natürlich wirkende Lavendelnote. Der Geruch erinnert ferner an schwarze Johannisbeeren, an geschnittene Brennesseln und an Königskraut. In Kompositionen werden mit dieser Substanz überraschende Effekte erzielt, die einerseits in einer natürlich wirkenden harmonischen Abrundung und andererseits einer Akzentuierung einer angenehmen, blumigen Note bestehen.

2,2-Dimethyl-6,8-nonadien-3-ol:
fruchtig (insbes. Citrus), erdig, blumig in Richtung Salbeiblüten. Überraschend ist bei diesem Alkohol der aldehydische Geruchscharakter. In Kompositionen ergibt der Citrus-Salbeikomplex eine originelle Note, die besonders in Herrenparfums geschätzt wird.

2,4,4,7-Tetramethyl-6,8-nonadien-3-on:
frisch, natürlich, an schwarze Johannisbeeren und an Bergamotteöl erinnernd.

3-Äthyl-4,7-dimethyl-6,8-nonadien-3-ol:
dieser tertiäre Alkohol zeichnet sich insbesondere durch seine Diffusion aus. Die Geruchsnote ist lavendelartig und wirkt sehr natürlich. In Kompositionen werden überraschende Effekte erzielt, indem der Alkohol diesen mehr Volumen verleiht und sie auf harmonische Weise abrundet.

2,3,4,4,7-Pentamethyl-6,8-nonadien-3-ol:
die Geruchsnote ist fruchtig, ein wenig erdig, an Grapefruit erinnernd, mit einem pilzigen und einem holzigen Unterton.

2,4,4,7-Tetramethyl-6,8-nonadien-3-on oxim:
sehr natürlich wirkende Note nach schwarzen Johannisbeeren, mit einem aussergewöhnlichen Haftvermögen.

Weitere bevorzugte Verbindungen I sind:
2,2,7-Trimethyl-6,8-nonadien-3-on,
2,2,7-Trimethyl-6,8-nonadien-3-ol,
2,2,3,7-Tetramethyl-6,8-nonadien-3-ol,
3,6-Dimethyl-5,7-octadien-2-ol,
5-Isopropyl-2,9-dimethyl-7,9-decadien-4-on,
2,4,4,7,8-Pentamethyl-6,8-nonadien-3-on,
2,4,4,8-Tetramethyl-6,8-nonadien-3-ol,
3,3,6-Trimethyl-5,7-octadien-2-on,
5-Äthyl-8-methyl-7,9-decadien-4-on,
3,3,6-Trimethyl-5,7-octadien-2-on-oxim,
5-Äthyl-8-methyl-7,9-decadien-4-on-oxim.

Die Verbindungen I verbinden sich mit zahlreichen bekannten Riechstoffingredienzien natürlichen oder synthetischen Ursprungs, wobei die Palette der natürlichen Rohstoffe sowohl leichtals auch mittel- und schwerflüchtige Komponenten, und diejenige der Synthetika Vertreter aus praktisch allen Stoffklassen umfassen kann, wie dies aus der folgenden Zusammenstellung ersichtlich ist:

– Naturprodukte, wie Baummoos-Absolue, Basilikumöl, Bergamotteöl, acetyliertes Cedernholzöl (z.B. Vertofix® IFF bzw. Cedartone® Givaudan), Eichenmoos, Kamillenöl, Galbanumöl, Geraniumöl, Jasmin Absolue und seine Substitute, Lavendelöl, Lavandinöl, Mastix Absolue, Corianderöl, Neroliöl, Patchouliöl, Petitgrainöl Paraguay, Elemiöl, Sandalholzöl, Vetiveröl, Ylang-Ylang-Öl, Zitronenöl, Wermutöl, Angelikasamenöl, Rosmarinöl, Mandarinenöl, Ysopöl.

– Alkohole, wie Linalool, Citronellol, Geraniol, natürliches Rhodinol, α-Terpineol, Phenyläthylalkohol, Phenylpropylalkohol, Zimtalkohol, 3-Methyl-5-(2′,2′,3′-trimethyl-cyclopent-3′-en-1′-yl)-pentan-2-ol (Sandelore® Givaudan), Dimethylbenzylcarbinol, Terpineol, Menthol, 2,2,8-Trimethyl-7-nonen-3-ol.

– Aldehyde, wie 3,5-Dimethyl-cyclohex-3-en-carboxaldehyd, Decanal, Methylnonylacetaldehyd, Hydroxycitronellal, α-Hexylzimtaldehyd, Cyclamenaldehyd, p-tert.Butyl-α-methyldihydro-zimt-aldehyd (z.B. Lilial® Givaudan), Citral, α-Amylzimtaldehyd, n-Undecen-10-al, n-Dodecanal.

– Ketone, wie α-Jonon, Acetylcedren, p-Methylacetophenon, Methyljonone, Allyljonon, p-Hydroxybenzylaceton.

– Ester, wie Cedrylacetat, cis-3-Hexenylacetat, cis-3-Hexenylbenzoat, Äthylacetoacetat, Linalylacetat, Geranylacetat, Terpenylacetat, Phenyläthylacetat, Styrallylacetat, p-tert.Butylcyclohexylacetat, 4-[4-Methyl-3-pentenyl]-cyclohex-3-en-1-yl-carbinylacetat (z.B. Myraldylacetat® Givaudan), Cinnamylformiat, Benzylacetat, Benzylsalicylat, Amylsalicylat, Isobutylsalicylat, Hexylsalicylat, Methyldihydrojasmonat, 3-Äthyl-1,1-dimethyl-cyclohex-3-en-2-carbonsäureäthylester (Givescone® Givaudan), Linalylanthranilat, 3-Äthyl-1,1,4-trimethyl-cyclohexen-3-en-2-carbonsäureäthylester (Myrascone® Givaudan).

– Lactone, wie γ-Nonalacton, γ-Decalacton, γ-Undecalacton, γ-Dodecalacton, Cumarin.

– verschiedene weitere, in der Parfümerie oft benützte Komponenten wie Moschus-Verbindungen (Ketonmoschus, 12-Oxahexadecanolid (z.B. Musk 174® Naarden), 1,1-Dimethyl-4-acetyl-6-tert.butylindan, Indol, p-Menthan-8-thiol-3-on, Eugenol, Acetaldehyd-propylphenyl-äthylacetal, Methyl-1-methyl-cyclododecyläther (z.B. Madrox® Givaudan), Phenylacetaldehyddimethylacetal (Viridine®), Cyclocitrylidenacetonitril, 8α, 12-Oxido-13,14,15,16-tetranorlabdan (Fixateur 404®).

Die Verbindungen der Formel I lassen sich in weiten Grenzen einsetzen, die beispielsweise von 0,1 (Detergentien) – 50% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 25%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Desodorantien, Detergentien, Tabak, etc.).

Die Verbindungen I können demgemäss bei der Herstellung von Kompositionen und – wie obige Zusammenstellung zeigt – unter Verwendung einer breiten Palette bekannter Riechstoffe, verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7.Auflage, Chapman und Hall, London, 1974 hervorgehend.

Die Verbindungen I sind im übrigen mit Vorteil auch – aus den Verbindungen II und III – unter Verwendung von Phasen-Transferagentien herstellbar, z.B. mittels Tricaprilylmethylammoniumchlorid («Aliquat 336» der Firma Henkel).

Beispiel 1
2,4,4-Trimethyl-6,8-nonadien-3-on

In einen 1 Liter-Rundkolben, der mit Rührer, Thermometer, Kühler und Tropftrichter versehen ist, gibt man 60 g pulverisiertes Kaliumhydroxyd und 180 ml trockenes Toluol. Man kühlt auf −10 °C ab, und lässt unter Rühren innerhalb 35 Minuten ein Gemisch von 30 g 1-Chlor-2,4-pentadien und 33 g Diisopropylketon zutropfen. Während der Zugabe wird die Temperatur unterhalb 0 °C gehalten, dann lässt man sie bis auf 20 °C ansteigen, und rührt das Reaktionsgemisch während 7 Stunden weiter. Man lässt 12 Stunden stehen und giesst dann den Kolbeninhalt auf 200 g zerstossenes Eis. Man trennt die gebildeten Schichten, und wäscht die organische Schicht mit Wasser neutral. Man dampft das Lösungsmittel ab, wobei 40 g Rohprodukt zurückbleiben, welche einer fraktionierten Destillation unterworfen werden.

Siedepunkt des reinen 2,4,4-Trimethyl-6,8-nonadien -3- ons: 54 °C / 0,75 mmHg; $n_D^{20}$ = 1,4711; $d_4^{20}$ = 0,8623. Geruch: lavendelartig, natürlich, erinnert an geschnittene Brennesseln und an schwarze Johannisbeeren.

Beispiel 2
2,2-Dimethyl-6,8-nonadien-3-on

Zu 76 g pulverisiertem Kaliumhydroxid in 230 ml trockenem Toluol wird unter Rühren bei einer Temperatur von 40 °C bis 45 °C innert 80 Minuten eine Mischung von 34 g 1- Chlor- 2,4-pentadien und 34,7 g 3,3-Dimethyl-butan-2-on (Pinakolin) zugetropft. Man kühlt nun auf Raumtemperatur ab und giesst den Kolbeninhalt auf 200 g zerstossenes Eis. Man trennt die gebildeten Schichten und wäscht die organische Schicht neutral. Man dampft das Lösungsmittel ab, wobei 56 g Rohprodukt anfallen, welche einer fraktionierten Destillation unterworfen werden. Siedepunkt des reinen 2,2-Dimethyl-6,8- nonadien -3-ons: 57 °C / 1,2 mmHg; $n_D^{20}$ = 1,4700; $d_4^{20}$ = 0,8618. Geruch: rosenartig, etwas camphrig, erinnert an Phenyläthyl-isobutyrat.

Beispiel 3
2,2-Dimethyl-6,8-nonadien-3-ol

In einen 1 Liter-Rundkolben, der mit Rührer, Thermometer, Kühler und Tropftrichter versehen ist, gibt man 6,8 g Lithiumaluminiumhydrid und 150 ml trockenen Äther. Zu dieser Suspension wird unter Rühren bei einer Temperatur von 10 °C innert 2 Stunden eine Lösung von 30 g 2,2-Dimethyl-6,8-nonadien-3-on in 300 ml trockenem Äther zugetropft. Nach der Zugabe wird das Reaktionsgemisch während 4 Stunden bei Rückflusstemperatur gehalten. Nach dem Abkühlen wird der Überschuss an Lithiumaluminiumhydrid mit 30 ml Äthylacetat zersetzt. Man giesst den Kolbeninhalt auf eiskalte 10%ige Schwefelsäure, trennt die gebildeten Schichten und wäscht die organische Schicht nacheinander mit Wasser, 8%iger Natriumhydrogencarbonatlösung und wiederum mit Wasser bis zur neutralen Reaktion. Nach dem Trocknen über Natriumsulfat wird der Äther abgedampft und der Rückstand (35 g) destilliert.

Siedepunkt des reinen 2,2-Dimethyl-6,8-nonadien-3-ols: 78 °C/2,5 mmHg; $n_D^{20}$ = 1,4809; $d_4^{20}$ = 0,8654. Geruch: fruchtig, nach Lavendel, etwas pilzartig.

Beispiel 4
2,2-Dimethyl-6,8-decadien-3-on

Nach dem Verfahren des Beispiels 2 erhält man aus 19,4 g 1-Chlor-2,4-hexadien und 17,4 g 3,3-Dimethyl-butan-2-on 30 g rohes 2,2-Dimethyl-6,8-decadien-3-on, welche einer fraktionierten Destillation unterworfen werden.

Siedepunkt des reinen Produkts: 49 °C/ 0,1 mmHg; $n_D^{20}$ = 1,4755; $d_4^{20}$ = 0,8613. Geruch: fruchtig, nach Birnen, an Geranylformiat erinnernd, rosenartig.

Beispiel 5
2,2-Dimethyl-6,8-decadien-3-ol

Nach dem Verfahren des Beispiels 3 erhält man durch Reduktion von 16,2 g 2,2-Dimethyl-6,8-decadien-3-on mit 3,4 g Lithiumaluminiumhydrid in 250 ml trockenem Äther 17 g Rohprodukt, welche durch fraktioniertes Destillieren 7,8 g reines 2,2-Dimethyl-6,8-decadien-3-ol ergeben.

Siedepunkt: 62 °C/0,2 mmHg; $n_D^{20}$ = 1,4861;

$d_4^{20}$ = 0,8703; Geruch: lavendelartig, pilzartig, an unreife Mandarinen erinnernd.

Beispiel 6
2,4,4,8-Tetramethyl-6,8-nonadien-3-on
Nach dem Verfahren des Beispiels 1 erhält man aus 58 g 1-Chlor-4-methyl-2,4-pentadien und 59 g Diisopropylketon 57 g rohes 2,4,4,8-Tetramethyl-6,8-nonadien-3-on, welche einer fraktionierten Destillation unterworfen werden.

Siedepunkt des reinen Produktes: 53 °C/0,25 mmHg; $n_D^{20}$ = 1,4770; $d_4^{20}$ = 0,8701; Geruch: lavendelartig, Bergamotte, Linalylacetatähnlich.

Beispiel 7
2,4,4,7-Tetramethyl-6,8-nonadien-3-on
In einen 2 Liter-Rundkolben, der mit Rührer, Thermometer, Kühler und Tropftrichter versehen ist, gibt man 264 g pulverisiertes Kaliumhydroxyd und 800 ml trockenes Toluol. Man kühlt auf −10 °C ab und tropft unter Rühren während 2 Stunden eine Mischung von 153,5 g 1-Chlor-3-methyl-2,4-pentadien und 151 g Diisopropylketon zu, wobei die Temperatur durch externe Kühlung unterhalb 0 °C gehalten wird. Das Reaktionsgemisch wird nach der Zugabe noch weitere 6 Stunden bei Zimmertemperatur gerührt. Man lässt 12 Stunden stehen und giesst dann den Kolbeninhalt auf 500 g zerstossenes Eis. Man trennt die gebildeten Schichten und wäscht die organische Schicht mit Wasser neutral. Das Lösungsmittel wird abgedampft, wobei 160 g Rohprodukt zurückbleiben, welche einer fraktionierten Destillation unterworfen werden.

Siedepunkt des reinen 2,4,4,7-Tetramethyl-6,8-nonadien-3-ons: 64 °C/0,3 mmHg; $n_D^{20}$ = 1,4790; $d_4^{20}$ = 0,8733; Geruch: fruchtig, nach Bergamotte, Ocimen, sehr natürlich wirkend.

Beispiel 8
2,2,7-Trimethyl-6,8-nonadien-3-on
Nach dem Verfahren des Beispiels 7 erhält man aus 116 g 1-Chlor-3-methyl-2,4-pentadien und 100 g 3,3-Dimethylbutan-2-on in Gegenwart von 200 g pulverisiertem Kaliumhydroxyd in 600 ml Toluol 141 g rohes 2,2,7-Trimethyl-6,8-nonadien-3-on, welche einer fraktionierten Destillation unterworfen werden.

Siedepunkt des reinen Produktes: 50 °C/0,1 mmHg; $n_D^{20}$ = 1,4763; $d_4^{20}$ = 0,8699; Geruch: lavendelartig, Ocimen, aber viel besser haftend als dieses.

Beispiel 9
4,7-Dimethyl-6,8-nonadien-3-on
Nach dem Verfahren des Beispiels 1 erhält man aus 58 g 1-Chlor-3-methyl-2,4-pentadien und 43 g Pentan-3-on (Diäthylketon) in Gegenwart von 50 g pulverisiertem Kaliumhydroxyd in 200 ml trockenem Pentan 51 g rohes 4,7-Dimethyl-6,8-nonadien-3-on, welche einer fraktionierten Destillation unterworfen werden.

Siedepunkt des reinen Produktes: 86 °C/6 mmHg; $n_D^{20}$ = 1,4788; $d_4^{20}$ = 0,8782; Geruch: lavendelartig, fruchtig, allylisch, an Grapefruit erinnernd.

Beispiel 10
3-Äthyl-4,7-dimethyl-6,8-nonadien-3-ol
In einem 1 Liter-Rundkolben, der mit Thermometer, Rührer, Kühler und Tropftrichter versehen ist, stellt man aus 9,4 g (0,39 Grammatom) Magnesium und 44,6 g (0,41 Mol) Äthylbromid in 210 ml trockenem Äther Äthylmagnesiumbromid her. Dazu gibt man die Lösung von 64,5 g (0,39 Mol) 4,7-Dimethyl-6,8-nonadien-3-on in 200 ml trockenem Äther. Nach beendigter Zugabe hält man das Reaktionsgemisch 3 Stunden bei Rückflusstemperatur und kühlt nachher in einem Eisbad ab. Der gebildete Magnesiumkomplex wird durch Zugabe einer Lösung von 30 g Ammoniumchlorid in 300 ml Wasser zersetzt. Man trennt die organische Phase ab und extrahiert die wässrige Schicht dreimal mit je 50 ml Äther. Die vereinigten organischen Phasen werden mit 5%iger Weinsäure, mit Wasser, mit einer 8%igen Natriumbicarbonatlösung und hierauf mit Wasser neutral gewaschen. Der Äther wird abgedampft und das Rohprodukt (72 g) destilliert.

Siedepunkt des reinen 3-Äthyl-4,7-dimethyl-6,8-nonadien-3-ols: 65 °C/0,2 mmHg; $n_D^{20}$ = 1,4947; $d_4^{20}$ = 0,8935; Geruch: sehr natürlich nach Lavendel, haftend und diffusiv.

Beispiel 11
5-Isopropyl-2,9-dimethyl-7,9-decadien-4-on
Nach dem Verfahren des Beispiels 1 erhält man aus 1-Chlor-4-methyl-2,4-pentadien und 2,6-Dimethyl-heptan-4-on das obige Keton; Sdp. = 72 °C/0,3 mmHg; $n_D^{20}$ = 1,4728. Geruch: linaloolartig mit einer Moschusnote, sehr natürlich wirkend.

Beispiel 12
2,4,4,7,8-Pentamethyl-6,8-nonadien-3-on
Nach dem Verfahren des Beispiels 1 erhält man aus 1-Chlor-3,4-dimethyl-2,4-pentadien und Diisopropylketon das obige Keton; Sdp. = 74 °C/0,22 mmHg; $n_D^{20}$ = 1,4795. Geruch: lavendelartig, ambrig, pilzig, etwas holzig.

Beispiel 13
2,2,7-Trimethyl-6,8-nonadien-3-ol
In einen 1 Liter-Rundkolben, der mit Rührer und Thermometer versehen ist, gibt man 41,8 g 2,2,7-Trimethyl-6,8-nonadien-3-on, 20 ml Wasser und 54 ml Äthanol. Zu diesem Gemisch gibt man unter Rühren portionenweise 6,1 g Natriumborhydrid, wobei man die Temperatur durch äussere Kühlung bei 25 °C hält. Nach der Zugabe wird noch während 3 Stunden bei 25 °C gerührt. Das Reaktionsgemisch wird mit einer Weinsäurelösung unter Kühlung neutralisiert und dann leicht angesäuert. Man nimmt in Äther auf, wäscht mit Wasser bis zur neutralen Reaktion und trocknet die ätherische Lösung über Natriumsulfat. Der Äther wird abgedampft und der Rückstand (41,4 g) destilliert.

Siedepunkt des reinen 2,2,7-Trimethyl-6,8-nonadien -3- ols: 59–60 °C/0,2 mmHg; $n_D^{20}$ = 1,4863. Geruch: fruchtig, nach Lavendelblüten, sehr natürlich wirkend.

Beispiel 14
2,4,4,8-Tetramethyl-6,8-nonadien-3-ol

Nach dem Verfahren des Beispiels 3 erhält man durch Reduktion von 2,4,4,8-Tetramethyl-6,8-nonadien-3-on mit Lithiumaluminiumhydrid in Diäthyläther den entsprechenden sekundären Alkohol mit dem Siedepunkt 89 °C/0,8 mmHg; $n_D^{20}$ = 1,4910. Geruch: lavendelartig, etwas balsamisch.

Beispiel 15
2,2,3,7-Tetramethyl-6,8-nonadien-3-ol

Nach dem Verfahren des Beispiels 10 erhält man aus 2,2,3,7-Tetramethyl-6,8-nonadien-3-on und Methylmagnesiumjodid den obigen Alkohol mit dem Siedepunkt 62 °C/0,05 mmHg; $n_D^{20}$ = 1,4910. Geruch: an Lavendelsamen erinnernd, sehr natürlich wirkend und gut haftend.

Beispiel 16

Nach dem Verfahren des Beispiels 10 erhält man aus 58,3 g 2,4,4,7-Tetramethyl-6,8-nonadien-3-on und Methylmagnesiumjodid (ex 7,2 g Magnesium) 57,9 g rohes 2,3,4,4,7-Pentamethyl-6,8-nonadien-3-ol, welche einer fraktionierten Destillation unterworfen werden.

Siedepunkt des reinen Produkts: 79 °C/ 0,3 mmHg; $n_D^{20}$ = 1,4983; $d_4^{20}$ = 0,9011. Geruch: fruchtig, an Grapefruit erinnernd, etwas erdig, staubig, holzig.

Beispiel 17

In einem mit Rührer, Thermometer und Kühler versehenen Rundkolben wird eine Lösung von 127 g 2,4,4,7-Tetramethyl-6,8-nonadien-3-on, 127 g Hydroxylamin-chlorhydrat, 127 ml Pyridin in 1,27 L Äthanol während 2 Stunden bei Rückflusstemperatur gehalten. Hierauf wird der grösste Teil des Äthanols abdestilliert. Nach dem Abkühlen wird auf Eiswasser gegossen und in Äther aufgenommen. Die Ätherlösung wird zuerst mit Wasser gewaschen, dann mit 5%iger Salzsäure (Eliminierung des Pyridinüberschusses), dann wieder mit Wasser bis zum Neutralpunkt. Nach dem Abdampfen des Äthers erhält man 109 g Rohprodukt, welche noch 86 g nicht reagiertes Keton enthalten. Das Keton wird abdestilliert. Der Rückstand besteht aus dem Oxim als Isomerengemisch und kann in Form einer 10%igen alkoholischen Lösung in der Parfümerie verwendet werden.

Ein Teil des Rückstandes wird durch Säulenchromatographie aufgetrennt: 17,8 g Rückstand werden in Toluol gelöst und auf 500 g Silicagel chromatographiert. Es werden 12 Fraktionen erhalten, wobei die Fraktionen 2 und 3 einerseits und 10 und 11 kristallin anfallen. Nach Umkristallisieren der Fraktionen 2 und 3 aus Methanol und Wasser erhält man eines der Oximisomeren mit dem Smp. 68–70 °C.

Beispiel 18

Nach dem Verfahren des Beispiels 1 erhält man aus 58,1 g 1-Chlor-2,4-pentadien und 45,2 g Isopropyl-methylketon in Gegenwart von 100 g pulverisiertem Kaliumhydroxyd in 300 ml Toluol 49,9 g rohes 3,3,6-Trimethyl-5,7-octadien-2-on, welche einer fraktionierten Destillation unterworfen werden.

Siedepunkt des reinen Produkts: 83 °C/ 6 mmHg; $n_D^{20}$ = 1,4815; $d_4^{20}$ = 0,8913. Geruch: ocimenartig, an gekochten Rhabarber erinnernd.

Beispiel 19

Nach dem Verfahren des Beispiels 1 erhält man aus 116,6 g Chlor-2,4-pentadien und 114,2 g Dipropylketon (Heptan-4-on) in Gegenwart von 200 g pulverisiertem Kaliumhydroxyd in 600 ml Toluol 91,9 g rohes 5-Äthyl-8-methyl-7,9-decadien-4-on, welche einer fraktionierten Destillation unterworfen werden.

Siedepunkt des reinen Produkts: 61 °C/ 0,2 mmHg; $n_D^{20}$ = 1,4770; $d_4^{20}$ = 0,8713. Geruch: fruchtig, gekochte Birnen, Zwetschgen.

Beispiel 20

Nach dem Verfahren des Beispiels 17 erhält man aus 33,2 g 3,3,6-Trimethyl-5,7-octadien-2-on 33,9 g rohes Oxim, welche einer fraktionierten Destillation unterworfen werden.

Siedepunkt des reinen Produkts: 73 °C/ 0,15 mmHg; $n_D^{20}$ = 1,5072; $d_4^{20}$ = 0,9339. Geruch: grün, natürlich, an Grapefruit und Sellerie erinnernd.

Beispiel 21

In einem 500 ml Rundkolben, der mit Rührer, Thermometer, Kühler und Tropftrichter versehen ist, löst man 26,3 g Hydroxylaminsulfat in 55 ml Wasser. Währenddem man 52 g 33%ige Natronlauge zugibt, hält man durch Kühlen die Temperatur bei 25 °C. Dann tropft man langsam bei Zimmertemperatur eine Lösung von 61 g 5-Äthyl-8-methyl-7,9-decadien-4-on in 120 ml Äthanol zu. Hierauf wird 4 Stunden bei Rückflusstemperatur gehalten. Nach dem Abkühlen wird auf Eiswasser gegossen, in Äther aufgenommen und neutral gewaschen. Das Lösungsmittel wird abdestilliert

und man erhält 64,3 g rohes Oxim, welche fraktioniert destilliert werden.

Siedepunkt des reinen Produkts: 95 °C/ 0,2 mmHg; $n_D^{20}$ = 1,4997; $d_4^{20}$ = 0,9141. Geruch: nach Selleriestengel, sehr natürlich wirkend, etwas nach Grapefruit, gut haftend.

Beispiel 22

Zu einer Parfumeriebase der Richtung Lindenblüte mit einem Gehalt an

| | Gewichtsteilen |
|---|---|
| Hydroxycitronellal | 150 |
| Linalool | 150 |
| α-Ionon | 100 |
| Lilial® (p-tert.Butyl-α-methylhydrozimtaldehyd) | 100 |
| Phenyläthylalkohol | 100 |
| Hexyl-salicylat | 50 |
| Linalylanthranilat | 50 |
| Galbanum synth. [10% DPG] (Dipropylenglykol) | 20 |
| römisches Kamillenöl (1% DPG) | 10 |
| Eugenol | 5 |
| Cumarin | 5 |
| DPG | 210 |
| | 950 |

werden 50 Gewichtsteile 2,4,4-Trimethyl-6,8-nonadien-3-on (Beispiel 1) zugefügt. Auf diese Weise wird die angenehme blumige, süsse Note, welche der Komposition einen abgerundeten, pudrigen Charakter verleiht, in willkommener Weise unterstrichen. Diese erhaltene neue Base eignet sich auf Grund ihrer Haftfestigkeit besonders zur Seifenparfümierung.

Beispiel 23

Einer Parfumerie-Base Richtung Zyklamen mit einem Gehalt an

| | Gewichtsteilen |
|---|---|
| 2-Äthyl-3,6,6-trimethyl-2-cyclohexencarbonsäureäthylester | 150 |
| Cyclamenaldehyd® (3-[p-Isopropylphenyl-2-methyl]-propionaldehyd) | 100 |
| Linalylacetat | 80 |
| Geraniol | 80 |
| Benzylacetat | 70 |
| Hydroxycitronellal | 50 |
| α-Hexylzimtaldehyd | 50 |
| Lilial® | 50 |
| Linalool | 50 |
| $C_{12}$-Aldehyd (10% DPG) | 20 |
| 2-Äthyl-6,6-dimethyl-2-cyclohexencarbonsäureäthylester | 20 |
| DPG | 230 |
| | 950 |

werden 50 Gewichtsteile 2,2-Dimethyl-6,8-nonadien-3-ol (Beispiel 3) zugegeben.

In der ursprünglichen Base herrschte die etwas rauhe Benzylacetatnote vor; diese wird durch die Zugabe blumiger, weicher und wärmer. Dadurch erhält die neue Komposition wesentlich mehr Volumen und mehr Diffusion.

Beispiel 24

Einer Parfumeriebase Richtung Fougère mit einem Gehalt an

| | Gewichtsteilen |
|---|---|
| Methyl-1-methyl-cyclododecyläther | 150 |
| 2,2,8-Trimethyl-7-nonen-3-ol | 150 |
| Bergamotteöl | 80 |
| Musc 174® (12-Oxahexadecanolid) | 70 |
| Hydroxycitronellal | 70 |
| Corianderöl | 50 |
| Patchouliöl | 50 |
| Baum-Moos (50% DPG) | 30 |
| Galbanumöl | 30 |
| Rhodinol | 30 |
| Isoraldein® (Isomethyl-α-jonon) | 30 |
| Petitgrainöl | 20 |
| Allyljonon | 20 |
| Elemiöl | 15 |
| Angelikasamenöl | 10 |
| Geraniumöl synth. | 10 |
| DPG | 85 |
| | 900 |

werden 50 Gewichtsteile 2,2-Dimethyl-6,8-nonadien-3-ol (Beispiel 3) zugegeben. Die Komposition erhält dadurch eine Citrusnote in Richtung Salbei und wirkt kräftiger und frischer.

Die Zugabe der neuen Substanz unterstreicht in der Komposition den Citrus-Salbeikomplex, welcher der Komposition eine ausserordentlich originelle Note verleiht. Die neue Komposition eignet sich sehr gut für moderne Herrenlinien.

Beispiel 25

Zu der Parfumerie-Base des Beispiels 16 (Richtung Lindenblüte) werden 50 Gewichtsteile 2,2-Dimethyl-6,8-decadien-3-ol (Beispiel 5) gegeben. Der Komposition wird dadurch ein aldehydischer Charakter verliehen, wodurch sie sich gut für das Parfümieren von kosmetischen Präparaten eignet.

Beispiel 26

Einer Komposition Richtung Cologne mit einem Gehalt an

| | Gewichtsteilen |
|---|---|
| Zitronenöl | 300 |
| Bergamotteöl | 200 |
| Neroliöl | 70 |
| Rosmarinöl | 70 |
| Petitgrainöl | 50 |
| Keton-Moschus® (4-tert.Butyl-3,5-dinitro-2,6-dimethylacetophenon) | 20 |
| Mandarinenöl | 15 |
| Hydroxycitronellal | 10 |
| Eugenol | 10 |
| Linalylanthranilat | 5 |
| DPG | 100 |
| | 850 |

werden 150 Gewichtsteile 2,4,4,8-Tetramethyl-6,8-nonadien-3-on (Beispiel 6) zugegeben. Nach dieser Zugabe wirkt die Komposition nun wesentlich «kräftiger», wobei die Citrusnote überraschenderweise in Richtung Grapefruit verändert

wird. Ausserdem tritt nun eine holzig-grüne Note hervor, welche der Komposition einen Vetivercharakter vermittelt und sie damit viel edler erscheinen lässt.

Beispiel 27

Parfumerie-Komplex in Richtung Salbei mit einem Gehalt an

| | Gewichtsteilen |
|---|---|
| 2,2,8-Trimethyl-7-nonen-3-ol | 200 |
| Methyldihydrojasmonat | 100 |
| Bergamotteöl | 100 |
| Methyl-1-methylcyclododecyläther | 100 |
| Ysopoel | 80 |
| Allyljonon | 30 |
| Corps Cassis® (p-Menthan-8-thiol-3-on) 1‰ | 5 |
| Fixateur 404® (8α, 12-Oxido-13,14,15,16-tetranorlabdan) | 5 |
| DPG | 280 |
| | 900 |

Die Base erinnert an Salbeikraut. Durch Zugabe von 100 Gewichtsteilen von 2,4,4,7-Tetramethyl-6,8-nonadien-3-on (Beispiel 7) entsteht ein neuartiger Komplex, welcher an Salbeiblüten erinnert und viel frischer und kräftiger als die ursprüngliche Base wirkt.

Beispiel 28

Die holzige angenehme Note der untenstehenden «Chypre»-Komposition Richtung Vetiver wird durch die Zugabe von 100 Gewichtsteilen 3-Äthyl-4,7-dimethyl-6,8-nonadien-3-ol (Beispiel 10) eindeutig verstärkt.

| | Gewichtsteile |
|---|---|
| 2-Äthyl-3,6,6-trimethyl-2-cyclohexen-carbonsäureäthylester | 100 |
| Isoraldein® | 100 |
| Geraniol | 60 |
| Methyldihydrojasmonat | 60 |
| Bergamotteöl | 50 |
| Keton-Moschus® | 35 |
| 2,2,8-Trimethyl-7-nonen-3-ol | 30 |
| Linalool | 30 |
| Benzylacetat | 20 |
| Methyl-1-methyl-cyclododecyläther | 20 |
| Patchouliöl | 15 |
| Sandalore® (3-Methyl-5-(2,2,3-trimethyl-cyclopent-3-en-1-yl) pentan-2-ol | 10 |
| Cyclocitryliden-acetonitril | 5 |
| Styrallylacetat | 5 |
| $C_{11}$-Aldehyd (10% DPG) | 5 |
| Undecalacton | 3 |
| p-Hydroxybenzylaceton | 2 |
| DPG | 350 |
| | 900 |

Es entsteht ein die Wirkstoffe verbindender Effekt, der die Base in ausserordentlicher Weise bereichert. Die Base kann ohne weiteres als fertiger Parfumextrakt verwendet werden.

Beispiel 29

Parfümerie Base Richtung Holz

| | Gewichtsteile |
|---|---|
| Methyl-1-methylcyclododecyläther | 150 |
| Vetivenylacetat | 100 |
| Sandela® Giv (3-Isocamphyl-(5)-cyclohexanol) | 100 |
| Linalool | 100 |
| Patchouliöl | 50 |
| Iron | 50 |
| Linalylacetat | 50 |
| Citronellol | 50 |
| Benzylacetat | 30 |
| Baum-Moos farblos absolut | 30 |
| α-Amylzimtaldehyd | 20 |
| Methylnonylacetaldehyd (10% DPG) | 20 |
| Eugenol | 20 |
| C-11-Aldehyd (10% DPG) | 10 |
| Ciste-öl Französisch | 10 |
| Sandalore® Giv | 10 |
| | 800 |

Gibt man zu dieser holzigen Parfumerie-Base 200 Teile 2,3,4,4,7-Pentamethyl-6,8-nonadien-3-ol, so wird diese in äusserst angenehmer Weise abgerundet. Die vorher allzu dominierende Vetivenylacetat-Note wird zugunsten einer angenehmen frisch-grünen Note verdrängt, die auch noch nach 24 Stunden vorherrscht.

Beispiel 30

Parfümerie-Cologne

| | Gewichtsteile |
|---|---|
| Bergamott-öl | 80 |
| 2-Äthyl-3,6,6-trimethyl-2-cyclo-hexen-1-yl-carbonsäureäthylester | 80 |
| Galaxolide® IFF (1,3,4,6,7,8-Hexahydro-4,6,6,7,8,8-hexamethyl-cyclopenta-γ-2-benzopyran) | 60 |
| Hydroxycitronellal | 60 |
| Methyl-1-methylcyclododecyläther | 60 |
| Bornylacetat | 40 |
| Keton-Moschus | 40 |
| Givescone® Giv | 20 |
| Petitgrainöl | 20 |
| Sandalore® Giv | 30 |
| Corps Cassis® Giv (p-Menthan-8-thiol-3-on) | 5 |
| Baum-Moos absolut | 5 |
| Dipropylenglykol | 450 |
| | 950 |

Es handelt sich hier um eine klassische Cologne-Base, welche durch den Zusatz von 100 Teilen 2,3,4,4,7-Pentamethyl-6,8-nonadien-3-ol organoleptisch enorm bereichert wird. Das Cologne wird völlig verändert: Durch ein Unterstreichen der Linalool-Cassis-Note wirkt diese sehr viel kräftiger, frischer und charaktervoller.

Gibt man dieselbe Menge 10%ige alkoholische Lösung der Oxime des 2,4,4,7-Tetramethyl-6,8-nonadien-3-ons, Rohprodukt des Beispiels 17 zu, so ist der Effekt ähnlich. Es wird aber in diesem Fall zusätzlich ein verblüffend natürlicher Coriander-Charakter festgestellt.

Beispiel 31

Blumiger Komplex

| | Gewichtsteile |
|---|---|
| Hydroxycitronellal | 700 |
| 2,4,4,7-Tetramethyl-6,8-nonadien-3-on | 60 |
| Hexylsalicylat | 40 |
| | 800 |

Durch Zugabe von 200 Teilen 5-Äthyl-8-methyl-7,9-decadien-4-on Oxim wird dieser blumige Komplex Richtung Lindenblüte verändert. Der vorher bloss grün-blumige Komplex weist nun auch noch – völlig überraschenderweise – eine pudrige Note auf.

Gibt man jedoch dieselbe Menge einer 10%igen alkoholischen Lösung der Oxime des 2,4,4,7-Tetramethyl-6,8-nonadien-3-ons (Rohprodukt des Beispiels 17) zu diesem Komplex, wird dieser sehr effektvoll Richtung würzig verändert. Die Komposition eignet sich besonders für Seifen und für Detergenzien-Basen.

Beispiel 32

Animalische Base

| | Gewichtsteile |
|---|---|
| Sandela® Giv | 100 |
| Methyl-1-methylcyclododecyläther | 100 |
| Acetylcedren | 100 |
| Patchouliöl | 50 |
| Benzylsalicylat | 40 |
| Linalylacetat | 40 |
| Myrrhenöl | 30 |
| Benzoe resinoid Siam | 30 |
| Äthylenbrassylat | 30 |
| Castoreum synth. | 30 |
| ω-Undecenal | 20 |
| Dodecanal | 20 |
| β-Ionon | 20 |
| p-Cresyl-phenylacetat | 5 |
| Indol | 5 |
| DPG | ad 1000 |

Gibt man zu dieser Base mit animalischem Charakter 100 Teile 5-Äthyl-8-methyl-7,9-decadien-4-on-oxim (Beispiel 21), so wird diese äusserst effektvoll unterstrichen, eine im Hinblick auf den Eigengeruch des Oxim überraschende Tatsache. Die Base wirkt nun viel wärmer und weist zudem mehr Volumen auf.

Beispiel 33

Würzige Base

| | Gewichtsteile |
|---|---|
| Benzylacetat | 100 |
| Hydroxycitronellal | 100 |
| Phenyläthylalkohol | 100 |
| Amylsalicylat | 100 |
| Patchouliöl | 80 |
| Ylang-öl | 50 |
| Eugenol | 50 |
| Linalylacetat | 60 |
| Keton-Moschus | 50 |
| Cedrylacetat | 30 |
| Epoxycedren | 30 |
| Acetyl-cedren | 30 |
| Cumarin | 30 |
| Krauseminzöl | 15 |
| Thymianöl | 15 |
| Zitronenöl | 5 |
| DPG | ad 1000 |

Durch Zugabe von 50 Teilen einer 10%igen Lösung der Oxime des 2,4,4,7-Tetramethyl-6,8-nonadien-3-ons (Beispiel 17) bringt man in diese Base der ursprünglich schwer-süssen Richtung eine minzig-würzige Note ein, die Gesamtnote hebt sich nun in eindrücklicher Weise von der Note der ursprünglichen Base ab. Der Komplex Krauseminze-Zitrone-Thymian wird hervorgehoben.

Auch die Zugabe von 100 Teilen 3,3,6-Trimethyl-5,7- octadien-2-on-oxim (Beispiel 20) bringt der Base viel mehr Leben, Frische. Die lavendelartige Eigennote der Substanz verbindet sich sehr vorteilhaft mit dem würzig-blumigen Element der Base zu einer Fougère-artigen, komplexen Wirkung.

**Patentansprüche**

1. Verbindungen der Formel

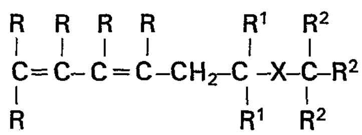 I

worin R, $R^1$ und $R^2$ Wasserstoff oder $C_{1-3}$-Alkyl darstellen, und X für 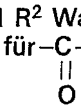, 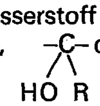 oder 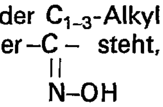 steht, wobei, wenn beide Symbole $R^1$ Wasserstoff sind, alle drei Symbole $R^2$ $C_{1-3}$-Alkyl darstellen, und wobei die Gesamtzahl der Kohlenstoffatome im Molekül 15 nicht überschreitet.

2. Verbindungen der Formel I gemäss Anspruch 1, worin X für 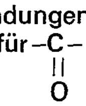 oder 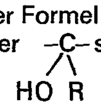 steht.

3. 2,2-Dimethyl-6,8-nonadien-3-ol.

4. 3-Äthyl-4,7-dimethyl-6,8-nonadien-3-ol.

5. 2,4,4-Trimethyl-6,8-nonadien-3-on.

6. 2,2,7-Trimethyl-6,8-nonadien-3-ol.

7. 2,2,7-Trimethyl-6,8-nonadien-3-on.

8. 2,2,3,7-Tetramethyl-6,8-nonadien-3-ol.

9. 2,4,4,7-Tetramethyl-6,8-nonadien-3-on.

10. 5-Äthyl-8-methyl-7,9-decadien-4-on.

11. 5-Äthyl -8- methyl -7,9- decadien -4- on-oxim.

12. 3,3,6-Trimethyl-5,7-octadien-2-on.

13. 3,3,6-Trimethyl-5,7-octadien-2-on-oxim.

14. 2,3,4,4,7-Pentamethyl-6,8-nonadien-3-ol.

15. 2,4,4,7- Tetramethyl -6,8- nonadien -3-on-oxim.

16. Eine Verbindung ausgewählt unter 2,2-Dimethyl-6,8-nonadien-3-on, 2,2-Dimethyl -6,8-decadien -3- ol, 2,2- Dimethyl -6,8- decadien-3-on, 4,7- Dimethyl -6,8- nonadien -3- on, 2,4,4,8-Tetramethyl -6,8- nonadien -3- on, 5- Isopropyl -2,9- dimethyl -7,9- decadien -4- on, 2,4,4,8-Tetramethyl -6,8- nonadien -3- ol, 2,4,4,7,8-Pentamethyl -6,8- nonadien -3- on.

17. Riechstoffkomposition, gekennzeichnet durch einen Gehalt an einer Verbindung der Formel I gemäss Anspruch 1.

18. Riechstoffkomposition, gekennzeichnet durch einen Gehalt an 2,4,4-Trimethyl-6,8-nonadien-3-on.

19. Riechstoffkomposition, gekennzeichnet durch einen Gehalt an 2,4,4,7-Tetramethyl-6,8-nonadien-3-on.

20. Riechstoffkomposition, gekennzeichnet durch einen Gehalt an 2,2,7-Trimethyl-6,8-nonadien-3-on.

21. Riechstoffkomposition, gekennzeichnet durch einen Gehalt an 2,2-Dimethyl-6,8-nonadien-3-ol.

22. Riechstoffkomposition, gekennzeichnet durch einen Gehalt an 3-Äthyl-4,7-dimethyl-6,8-nonadien-3-ol.

23. Riechstoffkomposition, gekennzeichnet durch einen Gehalt an 2,2,7-Trimethyl-6,8-nonadien-3-ol.

24. Riechstoffkomposition, gekennzeichnet durch einen Gehalt an 2,2,3,7-Tetramethyl-6,8-nonadien-3-ol.

25. Riechstoffkomposition, gekennzeichnet, durch einen Gehalt an 5-Äthyl-8-methyl-7,9-decadien-4-on.

26. Riechstoffkomposition, gekennzeichnet durch einen Gehalt an 5-Äthyl-8-methyl-7,9-decadien-4-on-oxim.

27. Riechstoffkomposition, gekennzeichnet durch einen Gehalt an 3,3,6-Trimethyl-5,7-octadien-2-on.

28. Riechstoffkomposition, gekennzeichnet durch einen Gehalt an 3,3,6-Trimethyl-5,7-octadien-2-on-oxim.

29. Riechstoffkomposition, gekennzeichnet durch einen Gehalt an 2,3,4,4,7-Pentamethyl-6,8-nonadien-3-ol.

30. Riechstoffkomposition, gekennzeichnet durch einen Gehalt an 2,4,4,7-Tetramethyl-6,8-nonadien-3-on-oxim.

31. Verfahren zur Herstellung von Verbindungen der Formel I gemäss Anspruch 1, dadurch gekennzeichnet, dass man eine Verbindung der Formel

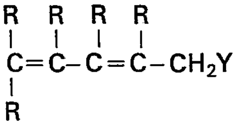

II

worin Y Halogen darstellt, mit einer Verbindung der Formel

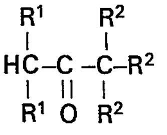

III

in Anwesenheit einer Base umsetzt, und gegebenenfalls ein erhaltenes Keton der Formel I a) zu einem sekundären Alkohol I reduziert oder b) durch Umsetzung mit einer Verbindung der Formel R Mg Y in einen tertiären Alkohol I überführt oder c) durch Umsetzung mit Hydroxylamin in ein Oxim I überführt.

32. Verwendung von Verbindungen der Formel I gemäss Anspruch 1 als Riechstoffe.

## Revendications

1. Composés de formule:

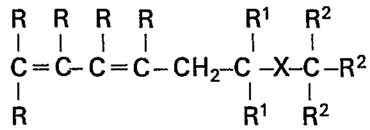

I

dans laquelle R, $R^1$ et $R^2$ représentent l'hydrogène ou des groupes alkyle en $C_1$–$C_3$ et X représente

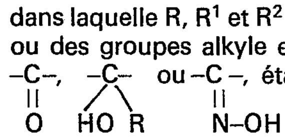

ou

étant spécifié que lorsque les deux symboles $R^1$ représentent l'hydrogène, les trois symboles $R^2$ représentent des groupes alkyle en $C_1$–$C_3$, et le nombre total des atomes de carbone de la molécule ne dépasse pas 15.

2. Composés de formule I selon la revendication 1, dans laquelle X représente

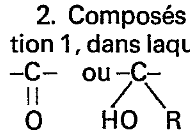

ou

3. Le 2,2- diméthyl -6,8- nonadiène -3- ol.

4. Le 3- éthyl -4,7- diméthyl -6,8- nonadiène -3- ol.

5. La 2,4,4-triméthyl-6,8-nonadiène-3-one.

6. Le 2,2,7-triméthyl-6,8-nonadiène-3-ol.

7. La 2,2,7-triméthyl-6,8-nonadiène-3-one.

8. Le 2,2,3,7-tétraméthyl-6,8-nonadiène-3-ol.

9. La 2,4,4,7 -tétraméthyl -6,8- nonadiène -3-one.

10. La 5-éthyl-8-méthyl-7,9-décadiène-4-one.

11. La 5-éthyl -8- méthyl -7,9- décadiène -4-onoxime

12. La 3,3,6-triméthyl-5,7-octadiène-2-one.

13. La 3,3,6-triméthyl -5,7- octadiène -2- on-oxime.

14. Le 2,3,4,4,7-pentaméthyl-6,8-nonadiène-3-ol.

15. La 2,4,4,7-tétraméthyl-6,8-nonadiène-3-on-oxime.

16. Un composé choisi parmi la 2,2-diméthyl-6,8-nonadiène-3-one, le 2,2-diméthyl-6,8-décadiène-3-ol, la 2,2-diméthyl-6,8-décadiène-3-one, la 4,7-diméthyl-6,8-nonadiène-3-one, la 2,4,4,8-tétraméthyl-6,8-nonadiène-3-one, la 5-isopropyl-2,9-diméthyl-7,9-décadiène-4-one, le 2,4,4,8 -tétraméthyl -6,8- nonadiène -3-ol, la 2,4,4,7,8-pentaméthyl-6,8-nonadiène-3-one.

17. Composition de parfum caractérisée en ce qu'elle contient un composé de formule I selon la revendication 1.

18. Composition de parfum caractérisée en ce qu'elle contient de la 2,4,4-triméthyl-6,8-nonadiène-3-one.

19. Composition de parfum caractérisée en ce

qu'elle contient de la 2,4,4,7-tétraméthyl-6,8-nonadiène-3-one.

20. Composition de parfum caractérisée en ce qu'elle contient de la 2,2,7-triméthyl-6,8-nonadiène-3-one.

21. Composition de parfum caractérisée en ce qu'elle contient du 2,2-diméthyl-6,8-nonadiène-3-ol.

22. Composition de parfum caractérisée en ce qu'elle contient du 3-éthyl-4,7-diméthyl-6,8-nonadiène-3-ol.

23. Composition de parfum caractérisée en ce qu'elle contient du 2,2,7-triméthyl-6,8-nonadiène-3-ol.

24. Composition de parfum caractérisée en ce qu'elle contient du 2,2,3,7-tétraméthyl-6,8-nonadiène-3-ol.

25. Composition de parfum caractérisée en ce qu'elle contient de la 5-éthyl-8-méthyl-7,9-décadiène-4-one.

26. Composition de parfum caractérisée en ce qu'elle contient de la 5-éthyl-8-méthyl-7,9-décadiène-4-on-oxime.

27. Composition de parfum caractérisée en ce qu'elle contient de la 3,3,6-triméthyl-5,7-octadiène-2-one.

28. Composition de parfum caractérisée en ce qu'elle contient de la 3,3,6-triméthyl-5,7-octadiène-2-on-oxime.

29. Composition de parfum caractérisée en ce qu'elle contient du 2,3,4,4,7-pentaméthyl-6,8-nonadiène-3-ol.

30. Composition de parfum caractérisée en ce qu'elle contient de la 2,4,4,7-tétraméthyl-6,8-nonadiène-3-on-oxime.

31. Procédé de préparation des composés de formule I selon la revendication 1, caractérisé en ce que l'on fait réagir un composé de formule:

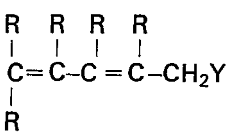

II

dans laquelle Y représente un halogène, avec un composé de formule:

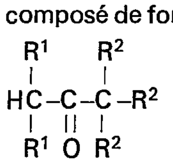

III

en présence d'une base, et le cas échéant, on réduit une cétone de formule I a) ainsi obtenue en un alcool secondaire I ou bien, b) on la convertit par réaction avec un composé de formule R Mg Y en un alcool tertiaire I ou bien c) on la convertit par réaction avec l'hydroxylamine en une oxime I.

32. Utilisation des composés de formule I selon la revendication 1 en tant que matières aromatiques.

## Claims

1. Compounds of the formula

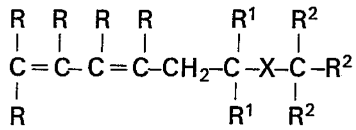

I

wherein R, $R^1$ and $R^2$ represent hydrogen or $C_{1-3}$-alkyl and X stands for

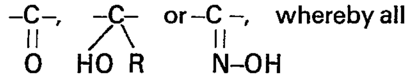

whereby all three symbols $R^2$ represent $C_{1-3}$-alkyl when both symbols $R^1$ are hydrogen and whereby the total number of carbon atoms in the molecule does not exceed 15.

2. Compounds of formula I in accordance with claim 1, wherein X stands for

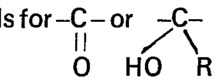

3. 2,2-Dimethyl-6,8-nonadien-3-ol.

4. 3-Ethyl-4,7-dimethyl-6,8-nonadien-3-ol.

5. 2,4,4-Trimethyl-6,8-nonadien-3-one.

6. 2,2,7-Trimethyl-6,8-nonadien-3-ol.

7. 2,2,7-Trimethyl-6,8-nonadien-3-one.

8. 2,2,3,7-Tetramethyl-6,8-nonadien-3-ol.

9. 2,4,4,7-Tetramethyl-6,8-nonadien-3-one.

10. 5-Ethyl-8-methyl-7,9-decadien-4-one.

11. 5-Ethyl -8- methyl -7,9- decadien -4- one oxime.

12. 3,3,6-Trimethyl-5,7-octadien-2-one.

13. 3,3,6-Trimethyl-5,7-octadien-2-one oxime.

14. 2,3,4,4,7-Pentamethyl-6,8-nonadien-3-ol.

15. 2,4,4,7-Tetramethyl-6,8-nonadien-3-one oxime.

16. A compound selected from 2,2-dimethyl-6,8-nonadien-3-one, 2,2-dimethyl-6,8-decadien-3-ol, 2,2-dimethyl-6,8-decadien-3-one, 4,7-dimethyl-6,8-nonadien-3-one, 2,4, 4,8-tetramethyl-6,8-nonadien-3-one, 5-isopropyl-2,9-dimethyl-7,9-decadien-4-one, 2,4,4,8-tetramethyl-6,8-nonadien-3-ol, 2,4,4,7,8-pentamethyl-6,8-nonadien-3-one.

17. An odorant substance composition, characterized by a content of a compound of formula I in accordance with claim 1.

18. An odorant substance composition, characterized by a content of 2,4,4-trimethyl-6,8-nonadien-3-one.

19. An odorant substance composition, characterized by a content of 2,4,4,7-tetramethyl-6,8-nonadien-3-one.

20. An odorant substance composition, characterized by a content of 2,2,7-trimethyl-6,8-nonadien-3-one.

21. An odorant substance composition, characterized by a content of 2,2-dimethyl-6,8-nonadien-3-ol.

22. An odorant substance composition, characterized by a content of 3-ethyl-4,7-dimethyl-6,8-nonadien-3-ol.

23. An odorant substance composition, characterized by a content of 2,2,7-trimethyl-6,8-nonadien-3-ol.

24. An odorant substance composition, characterized by a content of 2,2,3,7-tetramethyl-6,8-nonadien-3-ol.

25. An odorant substance composition, characterized by a content of 5-ethyl-8-methyl-7,9-decadien-4-one.

26. An odorant substance composition, characterized by a content of 5-ethyl-8-methyl-7,9-decadien-4-one oxime.

27. An odorant substance composition, characterized by a content of 3,3,6-trimethyl-5,7-octadien-2-one.

28. An odorant substance composition, characterized by a content of 3,3,6-trimethyl-5,7-octadien-2-one oxime.

29. An odorant substance composition, characterized by a content of 2,3,4,4,7-pentamethyl-6,8-nonadien-3-ol.

30. An odorant substance composition, characterized by a content of 2,4,4,7-tetramethyl-6,8-nonadien-3-one oxime.

31. A process for the manufacture of compounds of formula I in accordance with claim 1, characterized in that a compound of the formula

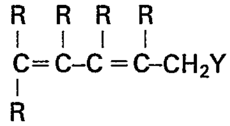

II

wherein Y represents halogen, is reacted with a compound of the formula

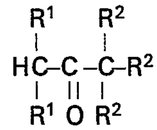

III

in the presence of a base and, if desired, a ketone of formula I obtained is a) reduced to a secondary alcohol I or b) converted into a tertiary alcohol I by reaction with a compound of the formula R Mg Y or c) converted into an oxime I by reaction with hydroxylamine.

32. The use of compounds of formula I in accordance with claim 1 as odorant substances.